# EUROPEAN PATENT SPECIFICATION

(11) **EP 3 108 941 B1**
(45) Date of publication and mention of the grant of the patent: **09.05.2018**
(21) Application number: 15173373.0
(22) Date of filing: 23.06.2015
(51) Int. Cl.: A61Q 19/00, A61Q 5/00, A61Q 5/12, A61Q 17/04, A61Q 19/02, A61Q 15/00, A61K 8/39, A61K 8/33, A61K 31/08, A61K 31/25, A61P 17/04

(54) **COMPOSITIONS COMPRISING POLYALKYLENE GLYCOL DERIVATIVES**
ZUBEREITUNGEN ENTHALTEND POLYALKYLENGLYKOL-DERIVATE
COMPOSITIONS CONTENANT DERIVÉES DE POLYALKYLÈNE GLYCOLE

(43) Date of publication of application: 28.12.2016
(73) Proprietor: Symrise AG, 37603 Holzminden (DE)
(72) Inventor: Schmaus, Gerhard, 37671 Höxter (DE); Lange, Sabine, 37603 Holzminden (DE)
(74) Representative: Fabry, Bernd

(56) References cited:
- WO-A1-2008/128892
- WO-A2-2012/143576
- MARTIN STEINHOFF ET AL: "Neurophysiological, Neuroimmunological, and Neuroendocrine Basis of Pruritus", JOURNAL OF INVESTIGATIVE DERMATOLOGY, vol. 126, no. 8, 1 August 2006 (2006-08-01), pages 1705-1718, XP055141873, ISSN: 0022-202X, DOI: 10.1038/sj.jid.5700231
- STEINHOFF M ET AL: "Proteinase-Activated Receptor-2 Mediates Itch: A Novel Pathway for Pruritus in Human Skin", JOURNAL OF NEUROSCIENCE, SOCIETY FOR NEUROSCIENCE, US, vol. 23, no. 15, 16 July 2003 (2003-07-16) , pages 6176-6180, XP002479656, ISSN: 0270-6474

## Description

### FIELD OF INVENTION

The present invention belongs to the area of cosmetics, and particularly so-called "cosmeceuticals" and refers to new actives for use in the treatment of itchy conditions of human skin and scalp.

### STATE OF THE ART

In about 10-20 percent of the population of industrial countries, with an increasing trend, atopy is to be observed. Atopy can manifest itself as dry itchy skin, predisposed atopic dermatitis or in severe disease states atopic dermatitis. The different severity states are associated with different severity of damaged skin and scalp barrier and skin is frequently itchy. Besides intrinsic, genetic factors, there are also extrinsic factors as for example environmental pollution, stress caused by UV , diverse chemical substances that may cause atopy. A too frequent usage of soap, shampoos or other hair care and/or colouring compositions, in general surfactants causing de-fattening and drying out of skin and scalp may also support the generation of an atopy and consequently itchy skin conditions.

There are only a view active compounds known, e.g. mast cell stabilizers like sodium chromoglycate, NK1 receptor antagonists like aprepitant or a number of plant extracts e.g. from oat, having an itch reducing action and that are already employed in the technical fields referred to, but alternatives nevertheless continue to be sought.

In connection with the invention described herein itch-reducing action is preferably to be understood as meaning the modulation, soothing, reduction, alleviating, elimination or prevention of itchy skin (for example, but not limited to, skin of the scalp), in particular that of the uncomfortable itch feeling that causes exhaustive scratching and in serious cases as a consequence injury of skin.

Persons skilled in the art have already addressed the problem of skin itch and have described, e.g. the skin itch-reducing properties of hydrocortisone or anti-histaminics. However, hydrocortisone, being considered as a potent anti-inflammatory agent, shows only poor itch-reducing activity while used for the treatment of conditions associated with dry scalp and/or skin, predisposed atopic skin or atopic dermatitis and skin and scalp barrier deficiencies. In this regard, it is to be noted that anti-inflammatory action does not necessarily go along with itch-reducing action. Regarding anti-histaminics, the scientific literature mentions that they are effective in case of e.g. insect bites, however they show only poor clinical efficacy in the treatment of itchy skin conditions associated with e.g. atopic dermatitis, predisposed atopic dermatitis and dry, itchy frequently winter skin, exposed to low and drying out temperature conditions.

According to scientific literature the itch-reducing action is predominantly based on soothing of the skin via inhibition of the PAR-2 receptor or, respectively, via the PAR-2 antagonistic action of compounds and mixtures described herein, preferably via the direct PAR-2 antagonistic action thereof. Particularly preferably, the (itch-reducing) action according to the invention is based on (direct) PAR-2 antagonistic activity, wherein the PAR-2 antagonist(s) (i.e. the compound(s) to be used according to the invention as described herein) mediate its/their effect(s) by binding to the active site or to allosteric site(s) on PAR-2 receptors, or interact at unique binding site(s), which are usually not involved in the biological regulation of the receptor's activity. A well-known approach for fighting or at least reducing itchy skin conditions is related to the topical administration of PAR-2 antagonists, which encompasses the additional benefit of strengthening the epidermal skin barrier against penetration of pollutants from the environment. Note that itch-reducing action does not necessarily refer to an anti-inflammatory effect.

The proteinase-activated receptor-2 (PAR-2) belongs to a family of four G-protein coupled receptors (GPCR's). PAR-2 signaling is irreversibly induced by various serine proteinases including mast cell tryptase, trypsine, kallikreins and others by cleavage and unmasking of the extracellular N-terminal domain, a mechanism termed tethered ligand activation (see literature: e.g. U.J.K. Soh et al., British Journal of Pharmacology, 160: 191-203, 2010 or S.E. Lee et al., Yonsei Med. J., 51 (6): 808-822, 2010). PARs are unique among GPCRs, in that their activation occurs by irreversible proteolytic cleavage of the N- terminus. Trypsin-like serine proteases cleave a cryptic ligand sequence in the N- terminus of PAR-2 (SKGR|SLIGR). In consequence the exposed N-terminus binds to the surface of the second extracellular loop of PAR-2 and triggers signaling cascades typically mediated by Gaq, Gα12/13 and Gaqi/o. Trypsin induced tethered ligand activation unmasks the peptide N-terminal sequence SLIGRL and e. g. Gaq-Protein triggered signaling cascade, and rise of intracellular calcium is induced. Thus, PAR-2 activation can also be triggered via application of small agonistic peptides like SLIGR and SLIGRL. Interaction of an activated PAR-2 with its accompanying heterotrimeric (αβγ)-G-protein leads to the nucleotide exchange (GDP to GTP) at the α-subunit of the G-protein. The activated G-protein dissociates into a Gα-GTP subunit and the βγ-subunit complex, which each can interact with different signal transducer proteins. The Gaq-GTP subunit can specifically interact and activate phospholipase C, which in turn catalyses the hydrolysis of membrane-bound phosphatidylinositol(4,5)-biphosphate [Ptdlns (4,5)P2] into diacylglycerol (DAG) and inositol(1,4,5)-triphosphate (IP3). IP3 formation results in release of Ca2+, which can be detected with fluorescence-sensitive dyes. At the same time IP3 also activates protein kinase C (PKC), which can be measured, e.g., at the level of gene transcription using appropriate reporter gene assays.

Skin exposure to exogenous applied PAR-2 activating proteases and agonistic peptides like SLIGR or SLIGRL can induce itch via PAR-2. A scientific study reported that mice, overexpressing epidermal KLK 7 displayed massive itchy behaviour *(*M. Steinhoff et al., J. Invest, Dermatology, Vol. 126, p. 1705-1718, 2006**).** Another scientific study reported that PAR-2 agonistic (activating) trypsin induced scratching behaviour in mice was inhibited by a PAR-2 antagonistic (inhibiting) peptide, suggesting a significant role of PAR-2 activation via serine proteases like tryptase in itch perception (R. Costa et al.; Br. J. Pharmacol., Vol. 154, p. 1094-1 103, 2008**).** Consequently, it is suggested that PAR- 2 antagonistic peptides might be a promising therapeutic tool for the prevention and/or treatment of itchy skin conditions thus helping to break the vicious itch-scratch cycle observed in itchy skin conditions. PAR-2 antagonistic peptides are described in the scientific literature as tool to investigate PAR-2 signalling pathways. However they are just occasionally described as a clinically efficient therapeutic tool for the prevention and or treatment of itchy skin conditions. It is commonly known that peptides are difficult to synthesize in large quantities. Furthermore, they are also known to have very poor ability to penetrate skin and, thus, consequently also to have only a poor ability to antagonise PAR-2 receptor signalling located on skin cells and sensory nerve endings and therefore only poor human clinical efficacy.

In the search for suitable agents it has to be remembered that the substances used should be toxicologically acceptable, tolerated well by the skin and stable (in particular in conventional cosmetic and/or pharmaceutical formulations), should preferably have the lowest possible intrinsic odour and the lowest possible intrinsic colour and be inexpensive to prepare. In accordance with the persistent trend towards natural active compounds, novel active compounds of natural, in particular plant origins are sought in particular.

For example, international patent application WO 2012 143576 A1 (SYMRISE) suggests compounds according to the following formula 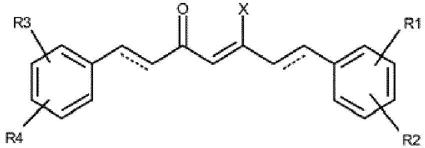 for the treatment of itchy conditions of skin. Although these compounds have been found to work well, they are expensive, difficult to incorporate into cosmetic formulation and are degradated or decomposed during storage, in particular at higher temperatures.

Therefore, the object of the present invention has been providing new compounds effective in the treatment of itchy conditions of skin and scalp, preferably at low concentrations, that are easy to prepare and to formulate and stable even when stored over weeks at elevated temperatures.

### DESCRIPTION OF THE INVENTION

A first object of the present invention is directed to a composition comprising or consisting of at least one polyalkylene glycol derivative according to general formula (I)

**R¹(OCH₂CHR²)ₙOR³** **(I)**

in which
- R¹: stands for hydrogen or a linear or branched alkyl group with 1 to 4 carbon atoms;
- R²: stands for hydrogen or methyl;
- n: represents an integer of from about 3 to about 20
- R³: stands for a linear or branched alkyl or alkenyl group with 6 to 18 carbon atoms and 0 or 1 to 3 double bonds or a -COR⁴ acyl group; and
- R⁴: stands for hydrogen, a linear or branched alkyl or alkenyl radical having 5 to 17 carbon atoms, or an alkali metal, an alkaline earth metal or ammonia
as a pharmaceutical active for
(i) use in preventing, treatment and/or cure of itchy conditions of human skin or scalp.

Surprisingly, it has been observed that polyalkylene glycol derivatives, for example PEG ethers, PEG esters and their combinations depending on the chain length of the hydrophobic moiety and the number of polyalkylene glycol units in the hydrophilic part represent highly effective PAR-2 antagonists and thus useful for preventing, treating, reducing and curing itchy conditions of human skin and scalp. The products are easy to obtain and can be formulated into cosmetic compositions without difficulties. Another major advantage is their high stability: even after storage over 6 weeks and 40 °C no degradation takes place.

### DEFINITIONS

In the context of this invention, itchy skin is preferably to be understood as meaning any change to the skin which induces sensorial malaise in humans and/or is characterized by the symptoms as dry, reddened or scaly skin. Skin itch can include, be caused or, respectively, be accompanied by phenomenological different skin states, such as delicate skin, sensitive skin, including sensitive scalp, easily injured skin, atopic skin, irritated skin or inflamed skin, which may manifest itself in an in each case higher severity in itch. In addition, itch is further on to be understood as meaning any change to the skin which induces the immediate desire to scratch. Itch is one of the most common disturbing skin conditions and can have high influence on life quality. Itch can be caused e.g. by rapid changes in temperature and/or wind, by specific systemic medication or by disturbed epidermal barrier. It is clearly on the one hand a neurological phenomenon with diverse receptors (e.g. histamine 1-4-, NK1-, TNF-alpha-, PAR-2-receptors) and respective mediators (histamines 1-4, Substance P, TNF-alpha, Tryptase, Trypsin and peptides like e.g. SLIGR and SLIGRL) being involved but on the other hand itch also clearly correlates with an impaired epidermal barrier. There is upcoming evidence that PAR-2 signalling is involved in e.g. atopic dermatitis, predisposed atopic dermatitis and dry skin, suffering from itchy skin. As described by Steinhoff et al (J. Neuroscience, Vol. 23(15), p.6176-6180, 2003**)** patients suffering from atopic dermatitis showed an up to fourfold increase of the PAR-2 agonist tryptase. Further on, the PAR-2 receptor was markedly enhanced on primary afferent nerve fibres in skin biopsies of atopic dermatitis patients.

In the context of the present invention "antagonistic activity" refers to a pharmaceutical and/or cosmetic active inhibition of the PAR-2 related bioactivity. According to a preferred embodiment, the compound(s) of formula 1 and/or salt(s) thereof or a composition according to the invention as described herein is/are used in an antagonistically effective amount, i.e. an amount sufficient to modulate, and preferably reduce by at least 20 percent, more preferably at least 50 percent, most preferably by at least 80 percent, the PAR-2 receptor activity, preferably measured as described in the working examples.

In the context of the present invention a pharmaceutical active has the meaning of a medicament.

In the context of the present invention the polyalkylene glycol moiety of the derivatives is abbreviated by PAG, while PEG stands for polyethylene glycol. A PEG-X compound has the meaning of polyethylene glycol derivative comprising a number of "X" PEG units.

### POLYALKYLENE GLYCOL ETHER DERIVATIVES

The polyalkylene glycol derivatives according to the present invention represent either ethers or esters of polyalkylene glycols (PAG) in general and polyethylene glycols (PEG) in particular. They are represented by formula (I)

**R¹(OCH₂CHR²)ₙOR³** **(I)**

in which
- R¹: stands for hydrogen or a linear or branched alkyl group with 1 to 4 carbon atoms, preferably for hydrogen;
- R²: stands for hydrogen or methyl, preferably for hydrogen;
- n: represents an integer of from about 3 to about 20, preferably for an integer of 5 to 12;
- R³: stands for a linear or branched alkyl or alkenyl group having 6 to 18 carbon atoms and 0 or 1 to 3 double bonds or a -COR⁴ acyl group; preferably for a linear or branched alkyl group having 8 to 15 carbon atoms, and
- R⁴: stands for hydrogen, a linear or branched alkyl or alkenyl radical having 5 to 17 carbon atoms, or an alkali metal, an alkaline earth metal or ammonia, preferably for a linear or branched alkyl group having 7 to 12 carbon atoms.
The derivatives are obtainable by standard methods of organic chemistry, as for example described in WO 2008128892 A1 (SYMRISE).

More particularly preferred are compositions comprising or consisting of at least one polyalkylene glycol derivative according to formula (Ia)

**H(OCH₂CH₂)ₙOR³** **(Ia)**

in which n represents an integer of from about 7 to 12 and R³ stands for a linear or branched alkyl group with 8 to 15 carbon atoms, and/or
compositions comprising or consisting of at least one polyalkylene glycol derivative according to formula (Ib)

**H(OCH₂CH₂)ₙO-COR⁴** **(Ib)**

in which n represents an integer of from about 3 to about 7 and R⁴ stands for a linear or branched alkyl group with 7 to 11 carbon atoms.

Suitable examples for polyalkylene glycol ethers according to formula (la) encompass:
- PEG-7 Octylether
- PEG-7 Nonylether
- PEG-7 Decylether
- PEG-7 Undecylether
- PEG-7 Dodecylether
- PEG-7 Tridecylether
- PEG-7 Tetradecylether
- PEG-8 Octylether
- PEG-8 Nonylether
- PEG-8 Decylether
- PEG-8 Undecylether
- PEG-8 Dodecylether
- PEG-8 Tridecylether
- PEG-8 Tetradecylether
- PEG-9 Octylether
- PEG-9 Nonylether
- PEG-9 Decylether
- PEG-9 Undecylether
- PEG-9 Dodecylether
- PEG-9 Tridecylether
- PEG-9 Tetradecylether
- PEG-10 Octylether
- PEG-10 Nonylether
- PEG-10 Decylether
- PEG-10 Undecylether
- PEG-10 Dodecylether
- PEG-10 Tridecylether
- PEG-10 Tetradecylether
- PEG-11 Octylether
- PEG-11 Nonylether
- PEG-11 Decylether
- PEG-11 Undecylether
- PEG-11 Dodecylether
- PEG-11 Tridecylether
- PEG-11 Tetradecylether
- PEG-12 Octylether
- PEG-12 Nonylether
- PEG-12 Decylether
- PEG-12 Undecylether
- PEG-12 Dodecylether
- PEG-12 Tridecylether
- PEG-12 Tetradecylether

The preferred polyalkylene glycol ether is PEG-9 Tridecylether.

Suitable examples for polyalkylene glycol esters according to formula (Ib) encompass:
- PEG-3 Octanoate
- PEG-3 Nonanoate
- PEG-3 Isononanoate
- PEG-3 Decanoate
- PEG-3 Undecanoate
- PEG-3 Dodecanoate
- PEG-4 Octanoate
- PEG-4 Nonanoate
- PEG-4 Isononanoate
- PEG-4 Decanoate
- PEG-4 Undecanoate
- PEG-4 Dodecanoate
- PEG-5 Octanoate
- PEG-5 Nonanoate
- PEG-5 Isononanoate
- PEG-5 Decanoate
- PEG-5 Undecanoate
- PEG-5 Dodecanoate
- PEG-6 Octanoate
- PEG-6 Nonanoate
- PEG-6 Isononanoate
- PEG-6 Decanoate
- PEG-6 Undecanoate
- PEG-6 Dodecanoate
- PEG-7 Octanoate
- PEG-7 Nonanoate
- PEG-7 Isononanoate
- PEG-7 Decanoate
- PEG-7 Undecanoate
- PEG-7 Dodecanoate

The preferred polyalkylene glycol ether is PEG-5 Isononanoate.

The by far preferred compositions refer to specific synergistic mixtures comprising or consisting of at least one PEG ether and at least one PEG ester, preferably a mixture of PEG-9 Tridecylether and PEG-5 Isononanoate.

The compositions according to the present invention may comprise or consist of
(i) ethers according to formula (la) and
(ii) esters according to formula (Ib),

wherein said components (a) and (b) are present in a ratio by weight of about 1:5 to about 5:1, preferably about 1:3 to about 3:1 and more preferably about 1:2 to about 2:1. These ratios are particular useful when using a mixture of PEG-9 Tridecylether and PEG-5 Isononanoate.

### COMPOSITIONS

Another object of the present invention refers to a cosmetic, a personal care or a pharmaceutical composition comprising
(a) at least one polyalkylene glycol derivative according to formula (la)

   **H(OCH₂CH₂)ₙOR**³ **(Ia)**

   in which n represents an integer of from about 7 to 12 and R³ stands for a linear or branched alkyl group with 8 to 15 carbon atoms; and
(b) at least one polyalkylene glycol derivative according to formula (Ib)

   **H(OCH₂CH₂)ₙO-COR⁴** **(Ib)**

   in which n represents an integer of from about 3 to about 7 and R⁴ stands for a linear or branched alkyl group with 7 to 12 carbon atoms.

More particularly said compositions comprise mixtures of
(a) PEG-9 Tridecylether and
(b) PEG-5 Isononanoate.

Preferably, the components (a) and (b) are present in a ratio by weight of about 1:5 to about 5:1, preferably about 1:3 to about 3:1 and more preferably about 1:2 to about 2:1, and also preferably they are added to the compositions in an amount of from about 0.01 to about 10 % b.w., more preferably in an amount of from about 0.05 to 5 % b.w. and most preferably in an amount of from about 0.1 to about 1 % b.w. - calculated on the final composition.

The compositions may represent a skin care, a personal care, a hair care, or a sun care composition.

### AUXILIARY AGENTS

In another preferred embodiment, the compositions may contain at least one auxiliary agent selected from the group consisting of anti-inflammatory agents, physiological cooling agents and skin care agents. These auxiliary agents may be present in amounts 0.01 to about 10 % b.w., more preferably in an amount of from about 0.05 to 5 % b.w. and most preferably in an amount of from about 0.1 to about 1 % b.w. - calculated on the final composition.

### Anti-inflammatory agents

Suitable anti-inflammatory agents may be selected from the group formed by:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

### Physiological cooling agents

Suitable physiological cooling agents may be selected from the group consisting of menthol, menthone glycerol acetal, menthone glyceryl ketal, menthyl lactate preferably I-menthyl lactate, in particular I-menthyl I-lactate), menthyl ethyl oxamate, substituted menthyl-3-carboxylic acid amides (e.g. menthyl-3-carboxylic acid N-ethylamide, N^{a}-(L- menthanecarbonyl)glycine ethyl ester, 2-isopropyl-N-2,3-trimethylbutanamide, substituted cyclohexanecarboxylic acid amides, 3-menthoxypropane-1,2-diol, 2- hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, monomenthyl glutarate, 2-mercaptocyclodecanone, menthyl 2-pyrrolidin-5-onecarboxylate, 2,3-dihydroxy-p- menthane, 3,3,5-trimethylcyclohexanone glycerol ketal, 3-menthyl 3,6-di- and - trioxaalkanoates, 3-menthyl methoxyacetate and icilin.

### Skin care agents

Suitable skin care agents encompass for example skin moisture retention regulators or skin repair agents, preferably selected from the group consisting of sodium lactate, urea and derivatives, glycerol, propylene glycol, 1,2-pentanediol, 1,2-hexanediol and 1,2- octanediol, alpha-bisabolol, apigenin, apigenin-7-glucoside, gingerols, shogaols, gingerdiols, dehydrogingerdiones, paradols, natural avenanthramides, non-natural avenanthramides, preferably dihydroavenanthramide D, boswellic acid, phytosterols, glycyrrhizin, glabridin and licochalcone A, preferably in the form of pure substances, collagen, elastin or hyaluronic acid, diacyl adipates, petrolatum, urocanic acid, lecithin, allantoin, panthenol, phytantriol, lycopene, (pseudo-)ceramides (preferably Ceramide 2, hydroxypropyl bispalmitamide MEA, cetyloxypropyl glyceryl methoxypropyl myristamide, N-(1-hexadecanoyl)-4-hydroxy- L-proline (1-hexadecyl) ester, hydroxyethyl palmityl oxyhydroxypropyl palmitamide), glycosphingolipids, cholesterol, phytosterols, chitosan, chondroitin sulfate, lanolin, lanolin esters, amino acids, vitamin E and derivatives (preferably tocopherol, tocopheryl acetate), alpha-hydroxy acids (preferably citric acid, lactic acid, malic acid) and derivatives thereof, mono-, di- and oligosaccharides, preferably glucose, galactose, fructose, mannose, laevulose and lactose, polysugars, such as beta -glucans, in particular 1,3-1,4-p-glucan from oats, alpha-hydroxy-fatty acids, triterpenic acids, such as betulic acid or ursolic acid, and algae extracts or single active compounds thereof.

### ADDITIVES

The preparations according to the invention may also contain further additives such as abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, antstatics, binders, buffers, carrier materials, chelating agents, cell stimulants, cleansing agents, care agents, depilatory agents, surface-active substances, deodorizing agents, antiperspirants, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing creams, ointments, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV-absorbing agents, UV filters, detergents, fabric conditioning agents, suspending agents, skin-tanning agents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, colourprotecting agents, pigments, anti-corrosives, aromas, flavouring substances, odoriferous substances, polyols, surfactants, electrolytes, organic solvents or silicone derivatives and the like as additional auxiliaries and additives.

### Surfactants

Preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆-₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

**Sorbitan esters.** Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

**Polyglycerol esters.** Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan GI 34), Polyglyceryl-3 Oleate, Diisostearoyl Polyglyceryl-3 Diisostearate (Isolan® PDI), Polyglyceryl-3 Methylglucose Distearate (Tego Care 450), Polyglyceryl-3 Beeswax (Cera Bellina®), Polyglyceryl-4 Caprate (Polyglycerol Caprate T2010/90), Polyglyceryl-3 Cetyl Ether (Chimexane NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

**Amphoteric emulsifiers.** Other suitable emulsifiers are amphboteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents and consistency factors

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents and rheology additives

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bisdimethylamino-1,3-propane, cationic guar gum such as, for example, Jaguar® CBS, Jaguar® C-17, Jaguar C-16 of Celanese, quaternized ammonium salt polymers such as, for example, Mirapol® A-15, Mirapol® AD-1, Mirapol® AZ-1 of Miranol and the various polyquaternium types (for example 6, 7, 32 or 37) which can be found in the market under the tradenames Rheocare® CC or Ultragel® 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlizing waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes and stabilizers

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, it is advantageous to combine compounds of formula (I) with active ingredients which penetrate into the skin and protect the skin cells from inside against sunlight-induced damage and reduce the level of cutaneous matrix metalloproteases. Preferred respective ingredients, so called arylhydrocarbon receptor antagonists, are described in WO 2007/128723, incorporated herein by reference. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan®HMS)
- salicylic acid-2-ethylhexyl ester (Neo Heliopan®OS)
- triethanolamine salicylate
- 4-isopropyl benzyl salicylate
- anthranilic acid menthyl ester (Neo Heliopan®MA)
- diisopropyl cinnamic acid ethyl ester
- p-methoxycinnamic acid-2-ethylhexyl ester (Neo Heliopan®AV)
- diisopropyl cinnamic acid methyl ester
- p-methoxycinnamic acid isoamyl ester (Neo Heliopan®E 1000)
- p-methoxycinnamic acid diethanolamine salt
- p-methoxycinnamic acid isopropyl ester
- 2-phenylbenzimidazole sulfonic acid and salts (Neo Heliopan®Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan®MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb®HEB)
- benzylidene malonate polysiloxane (Parsol®SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul®T150).

Broadband filters which are preferably combined with one or more compounds of formula (I) in a preparation according to the present invention are selected from the group consisting of
- 2-ethylhexyl-2-cyano-3,3-diphenyl acrylate (Neo Heliopan®303)
- ethyl-2-cyano-3,3'-diphenyl acrylate
- 2-hydroxy-4-methoxybenzophenone (Neo Heliopan®BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 2,2'-methylene bis-(6-(2H-benzotriazol-2-yl)-4-1,1,3,3-tetramethylbutyl) phenol) (Tinosorb®M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb®S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl)phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine.

The compositions can comprise further typical detergent and cleansing composition ingredients such as UV-A filters filters which are preferably combined with one or more compounds of formula (I) in a preparation according to the present invention are selected from the group consisting of
- 4-isopropyl dibenzoyl methane
- terephthalylidene dibornane sulfonic acid and salts (Mexoryl®SX)
- 4-t-butyl-4'-methoxydibenzoyl methane (avobenzone) / (Neo Heliopan®357)
- phenylene bis-benzimidazyl tetrasulfonic acid disodium salt (Neo Heliopan®AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul® A Plus)
- indanylidene compounds in accordance with DE 100 55 940 A1 (= WO 2002 038537 A1)

The compositions can comprise further typical detergent and cleansing composition ingredients such as UV filters which are more preferably combined with one or more compounds of formula (I) in a preparation according to the present invention are selected from the group consisting of
- p-aminobenzoic acid
- 3-(4'-trimethylammonium) benzylidene bornan-2-one methyl sulfate
- salicylic acid homomenthyl ester (Neo Heliopan®HMS)
- 2-hydroxy-4-methoxybenzophenone (Neo Heliopan®BB)
- 2-phenylbenzimidazole sulfonic acid (Neo Heliopan®Hydro)
- terephthalylidene dibornane sulfonic acid and salts (Mexoryl®SX)
- 4-tert-butyl-4'-methoxydibenzoyl methane (Neo Heliopan®357)
- 3-(4'-sulfo)benzylidene bornan-2-one and salts
- 2-ethylhexyl-2-cyano-3,3-diphenyl acrylate (Neo Heliopan®303)
- N-[(2 and 4)-[2-(oxoborn-3-ylidene) methyl]benzyl] acrylamide polymer
- p-methoxycinnamic acid-2-ethylhexyl ester (Neo Heliopan®AV)
- p-aminobenzoic acid ethyl ester (25 mol) ethoxylated (INCI name: PEG-25 PABA)
- p-methoxycinnamic acid isoamyl ester (Neo Heliopan®E1000)
- 2,4,6-trianilino-(p-carbo-2'-ethylhexyl-1'-oxy)-1,3,5-triazine (Uvinul®T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan®MBC)
- 3-benzylidene camphor
- salicylic acid-2-ethylhexyl ester (Neo Heliopan®OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb®M)
- phenylene bis-benzimidazyl tetrasulfonic acid disodium salt (Neo Heliopan®AP)
- 2,4-bis-[{(4-(2-ethylhexyloxy)-2-hydroxy}phenyl]-6-(4-methoxyphenyl)-1,3,5-triazine (Tinosorb®S)
- benzylidene malonate polysiloxane (Parsol®SLX)
- menthyl anthranilate (Neo Heliopan®MA)
- 2-(4-diethylamino-2-hydroxybenzoyl) benzoic acid hexyl ester (Uvinul® A Plus)
- indanylidene compounds in accordance with DE 100 55 940 (= WO 02/38537).

Advantageous primary and also secondary sun protection factors are mentioned in WO 2005 123101 A1. Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) so that the formulation according to the invention has a light protection factor of greater than or equal to 2 (preferably greater than or equal to 5). Such formulations according to the invention are particularly suitable for protecting the skin and hair.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### Actives modulating skin and/or hair pigmentation

Preferred active ingredients for skin and/or hair lightening are selected from the group consisting of: kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates (preferably one or more cyclohexyl carbamates disclosed in WO 2010/122178 and WO 2010/097480), sulfurcontaining molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract .

Preferred skin lighteners as component (b) are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases") as described for example in EP 0 584 178, tetrasubstituted cyclohexene deriva-tives as described for example in WO 2005/032501, isoprenoids as described in WO 2005/102252 and in WO 2006/010661, melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, microalgae extracts, in particular Isochrysis galbana, trehalose, erythru-lose and dihydroxyacetone. Flavonoids which bring about skin and hair tinting or brown-ing (e.g. quercetin, rhamnetin, kaempferol, fisetin, genistein, daidzein, chrysin and api-genin, epicatechin, diosmin and diosmetin, morin, quercitrin, naringenin, hesperidin, phloridzin and phloretin) can also be used.

The amount of the aforementioned examples of additional active ingredients for the modulation of skin and hair pigmentation (one or more compounds) in the products according to the invention is then preferably 0.00001 to 30 wt.%, preferably 0.0001 to 20 wt.%, particularly preferably 0.001 to 5 wt.%, based on the total weight of the preparation.

### Anti-ageing actives

In the context of the invention, anti-ageing or biogenic agents are, for example antioxidants, matrix-metalloproteinase inhibitors (MMPI), skin moisturizing agents, glycosaminglycan stimulkators, anti-inflammatory agents, TRPV1 antagonists and plant extracts.

**Antioxidants.** Suitable antioxidants encompass amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gammalinoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.

If vitamin E and/or derivatives thereof are used as the antioxidant(s), it is advantageous to choose their concentrations from the range from about 0.001 to about 10 % b.w. based on the total weight of the formulation. If vitamin A or vitamin A derivatives or carotenes or derivatives thereof are used as the antioxidant(s), it is advantageous to choose their concentrations from the range from about 0.001 to aout 10 % b.w. based on the total weight of the formulation.

**Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02 069992 A1 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1, incorporated herein by reference) as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators.** Preferred compositions comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**Anti-inflammatory agents.** The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1, are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenan-thramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide (CO2), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1, or indirect modulators of TRPV1 by an activation of the µ-receptor, e.g. acetyl tetrapeptide-15, are preferred.

**Desquamating agents.** The compositions may also contain desquamating agents (component b5) in amounts of about 0.1 to about 30 % b.w. preferably about 0.5 to about 15 % b.w., particularly preferably about 1 to about 10 % b.w. based on the total weight of the preparation. The expression "desquamating agent" is understood to mean any compound capable of acting:
- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine®, prickly pear extracts such as those marketed under the name Exfolactive® by the company SILAB, or Phytosphingosine SLC® (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, those described in the **documents** FR 2570377 A1, EP 0199636 A1, EP 0325540 A1, EP 0402072 A1, chestnut or prickly pear extracts, in particular marketed by SILAB; reducing compounds such as cysteine or cysteine precursors.

Desquamating agents which can be used are also nicotinic acid and its esters and nicotinamide, also called vitamin B3 or vitamin PP, and ascorbic acid and its precursors, as described in particular in application EP 1529522 A1**.**

**Anti-cellulite agents.** Anti-cellulite agents and lipolytic agents are preferably selected from the group consisting of those described in WO 2007/077541, and beta-adrenergic receptor agonists such as synephrine and its derivatives, and cyclohexyl carbamates described in WO 2010/097479. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents.** Formulations and products according to the present invention may also comprise one or more fat enhancing and/or adipogenic agents as well as agents enhancing or boosting the activity of fat enhancing agents. A fat enhancing agent is for example hydroxymethoxyphenyl propylmethylmethoxybenzofuran (trade name: Sym3D®).

### Hair growth activators or inhibitors

Formulations and products according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/nd hydrolysates from rice or wheat.

Alternatively, formulations and products according to the present invention may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, San-guisorba officinalis, Calendula officinalis, Hamamelis virginiana, Arnica montana, Salix alba, Hypericum perforatum or Gymnema sylvestre.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronel-lol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethylpentyl)-2-(1H)-pyridinone monoethanolamine salt), Baypival (Climbazole), Keto-conazol® (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon® UD (protein/undecylenic acid condensate), zinc pyrithione, aluminium pyrithione and magnesium pyrithione/dipyrithione magnesium sulfate.

### Carriers and hydrotropes

Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, ▪-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Farbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

### Preparations

Preferred compositions according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation.

### EXAMPLE 1

### In vitro PAR-2 assay

**Assay Principle:** A cellular in vitro assay was developed with human embryonic kidney 293 cells (HEK293) endogenously expressing PAR-2. The PAR-2 activating protease trypsin (EC₅₀: ^{∼}1nM) was used as an agonistic stimulus with subsequent Fluo-4 dependent intracellular calcium detection (calcium imaging). Polyethyene glycol esters and polyethylene glycol ethers, significantly reducing the trypsin mediated PAR-2 activation, were further evaluated for dose response effects and for determination of IC₅₀ values of PAR-2 antagonistic activity.

A cell-based calcium assay was applied for the identification of PAR-2 antagonistic effects of polyethylene glycol esters and polyethylene glycol ethers. Functional modulation of PAR-2 by the tested compounds was measured and quantified using the calcium sensitive fluorescent probe Fluo-4 AM on a fluorescence microplate reader. Activation of PAR-2 by an agonist (trypsin) led to an increase in the intracellular calcium concentration and thus an increase in fluorescence intensity. Inhibition of the PAR-2 related bioactivity by an antagonist reduced an agonist-evoked increase in fluorescence intensity significantly or preferably blocked the agonist-evoked signal completely.

**Procedure:** Human embryonic kidney 293 cells (HEK293) endogenously expressing PAR-2 were cultured in DMEM (high glucose) supplemented with tetracycline-free FCS (10%v/v), L-glutamine (4mM), blasticidin (15µg/ml), zeocin (100µg/ml) and puromycin (2µg/ml) in a water-saturated atmosphere at 37°C and 5% CO₂.

Cells were seeded onto 96-well clear-bottom black-walled assay plates at a density of 45.000 cells per well in 100µl of cell culture medium. Intracellular calcium increase in the living cells due to receptor activation was monitored 24 h later using the calcium sensitive fluorescent probe Fluo-4 AM on a fluorescence microplate reader (FlexStation® system, Molecular Devices). Therefore 100 µl Krebs-HEPES (KH) buffer (118mM NaCl; 4.7mM KCI; 1.3mM CaCl₂; 1.2mM MgSO₄; 1.2mM KH₂PO₄; 4.2mM NaHCO₃; 10mM Hepes, pH 7.4) supplemented with sulfinpyrazone (250µM) and Fluo-4 AM (4µM) were added and the cells were incubated for an additional hour in a water-saturated atmosphere at 37°C and 5% CO₂.

**Measurement of antagonistic activity:** Medium was replaced with 150µl KH buffer supplemented with sulfinpyrazone (250µM). Subsequently, 50µl KH buffer supplemented with the screening compounds or control substances were added and the cells were incubated for 10 min. under assay conditions in the microplate reader. Changes in fluorescence were recorded at 37°C after addition of 50µl KH buffer supplemented with the PAR-2 agonist trypsin (final concentration of 4nM under measurement conditions).

**Analysis:** Calcium mobilization was quantified as the change of peak fluorescence (ΔF) over the baseline level (F). The data was analysed with the software of the microplate reader. Potential PAR-2 antagonists were tested in an effective range of 1-100 µM for their capacity to reduce the trypsin-evoked signal.

### EXAMPLE 1.1

PAR-2 antagonistic activity of PEG-5 Isononanoate in cell based fluorescent analysis.
In the presence of 4 nM trypsin the sample tested for PAR-2 antagonism delivered a dose dependent inhibition with an IC₅₀ of 100µM.

### EXAMPLE 1.2

PAR-2 antagonistic activity of PEG-9 Tridecylether in cell based fluorescent analysis.

In the presence of 4 nM trypsin the sample tested for PAR-2 antagonism delivered a dose dependent inhibition with an IC₅₀ of 65µM.

### EXAMPLE 1.3

Synergistic PAR-2 antagonistic activity in cell based fluorescent analysis of a composition according to this invention, comprising PEG-5 Isononaoate and PEG-9 Tridecylether in a ratio of 1:2 (w/w)

In the presence of 4nM trypsin the combination tested for PAR-2 antagonism delivered a dose dependent inhibition with an IC₅₀ of 26µM.

The results of the trials are compiled in the following **Table 1.**

**Table 1**

| PAR-2 antagonistic activity of a composition comprising PEG-5 Isononanoate and PEG-9 Tridecylether in a ratio of 1:2; w/w | |
|---|---|
| **Compound** | **PAR-2 IC₅₀ [µM]** |
| PEG-5 Isononanoate | 100 |
| PEG-9 Tridecylether | 65 |
| PEG-5 Isononanoate/PEG-9 Tridecylether (1:2; w/w) | 26 |

The Synergy Index (SI) was calculated according to Kull's equation with regard to the IC₅₀ values. Synergy in a composition exists when the synergy index is less than 1, means the lower the SI the greater the synergy. SI for PEG-5 Isononanoate/PEG-9 Tridecylether (1:2; w/w) was calculated being 0.35.

The significantly lower IC₅₀ value of 26 µM of the combination comprising PEG-5 Isononanoate and PEG-9 Tridecylether at a ratio of 1:2 (w/w) delivered the unequivocal proof that the PAR-2 antagonistic activity of the composition is synergistically increased versus the single compounds PEG-5 Isononanoate and PEG-9 Tridecylether, respectively.

Advanced validation analysis on PAR-2 specificity of identified antagonists was conducted with HEK293-sh20 cells stably expressing a non-specific random shRNA (sh20) versus HEK293-sh65 cells stably overexpressing a specific, PAR-2 targeting shRNA (sh65). polyethylene glycol esters and polyethylene glycol ethers and compositions comprising polyethylene glycol esters and polyethylene glycol ethers according to the invention proved to be highly specific PAR-2 antagonists as a significant shift in IC₅₀ values towards lower concentrations regarding PAR-2 inhibition in HEK293-sh65 cells compared to the HEK293-sh20 cells was observed. This clearly indicated that polyethylene glycol esters, polyethylene glycol ethers and compositions comprising polyethylene glycol esters and polyethylene glycol ethers exhibit their skin soothing and anti-itch activity via highly specific inhibition of PAR-2 and downstream signaling pathways.

### EXAMPLE 3

### Clinical trials

The aim of the single blinded clinical cross-over study was to evaluate and to compare the efficacy, skin compatibility and tolerability of two exemplary anti-itch shampoos (SL15VS021-2 comprising 2% of PEG-5 Isononanoate/PEG-9 Tridecylether ratio 1:2; w/w %, versus placebo shampoo SL15VS021-1) after a single application and after repeated applications. It was carried out in a temperature and humidity-controlled room (24 +/- 2°C; 50 +/-10% R.U.). 20 volunteers (13 males and 7 females) suffering from itching on the scalp were selected for the study.

They were asked not to use any anti-itching treatment on the scalp for the whole duration of the test. Moreover, volunteers were asked to wash their hair 48 hours before each visit and to refrain from the application of any styling products.

The volunteers underwent a 7-day wash-out period during which they were requested to use a standard shampoo, code SL15VS021-1, before starting the test.

Each volunteer tests both shampoos, one by one, according to a randomization chart. The assignment of subject number and subsequent placement on the randomization chart were made in order of appearance at the study centre on the first day. The products were given in anonymous containers which did not provide any information about the treatment.

Firstly, at the baseline the volunteers were clinically examined and scored for the degree of *dandruff, seborrhoea* and *erythema.* Furthermore, they were asked about the sensations of *burning* and *itching.* The evaluations of dandruff, seborrhoea, erythema, burning and itching were performed on the basis of the following 4-point clinical scale: 0 *= absent, 1 = mild, 2 = moderate, 3 = severe.*

Then, the volunteers were asked to score the *decrease in itching sensation after 1, 5, 15 and 30 minutes* from the first use of the shampoo, according to the scale: *0 = no decrease, 1 = poor decrease, 2 = moderate decrease, 3 = good decrease.*

Finally, each volunteer was given the assigned shampoo to be used at least three times a week for 1 week. The following check was performed at the end of the 7-day treatment.

The volunteers underwent an additional 7-day wash-out period during which they were asked to use the standard shampoo, code SL15VS021-1. After the wash-out period the subjects came back to the laboratory to receive the second shampoo according to the same procedure described above: basal visit, short term evaluation of the itching decrease, 7-day treatment with the product and final clinical assessment.

Moreover, the *overall tolerability* of each shampoo was clinically scored, at the end of the treatment, according to the scale: *1 = poor tolerability; 2 = moderate tolerability; 3 = good tolerability.* The compositions are shown in **Table 2.**

**Table 2**

| Shampoo formulation | | | |
|---|---|---|---|
| **Shampoo Formulation** | | **w/w %** | |
| **Raw Material** | | **SL15VS021-2** | **SL15VS021-1 Placebo + Standard Shampoo** |
| **A.** | Sodium Laureth Sulfate, Lauryl Glycoside (60% AS) | 21.0 | 21.0 |
| | Citric Acid | 0.2 | 0.2 |
| | Disodium EDTA | 0.1 | 0.1 |
| **B.** | Water (Aqua) | Ad 100 | Ad 100 |
| | Sodium Benzoate | 0.5 | 0.5 |
| | Hydroxyacetophenone | 0.5 | 0.5 |
| | Cocoamidoproyl Betaine (38% AS) | 7.0 | 7.0 |
| **C.** | PEG-5 Isononanoate/PEG-9 Tridecylether, ratio 1:2 | **2.0** | - |
| | SUM | 100.0 | 100.0 |
| | pH value | 5.9 | 5.9 |

### Results of short term evaluation

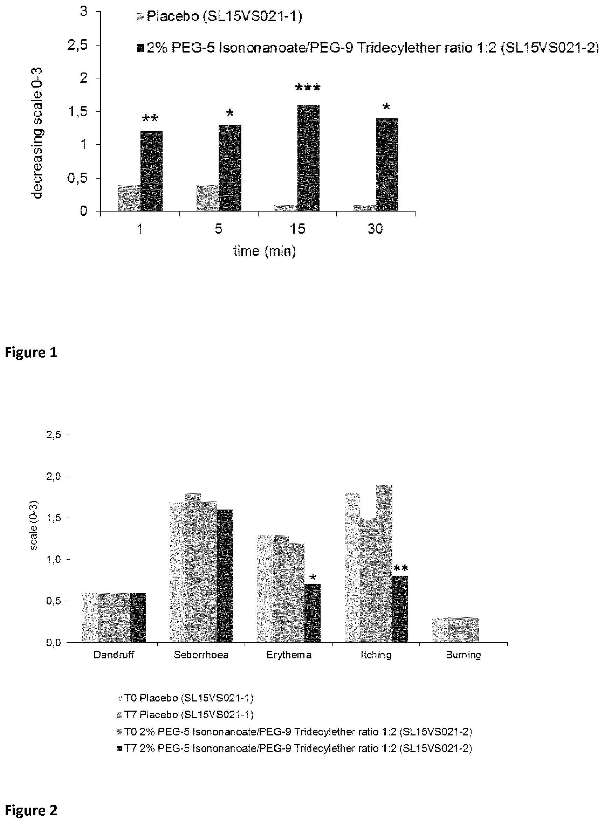

The comparison between the two products showed that shampoo SL15VS021-2 (with 2% of PEG-5 Isononanoate/PEG-9 Tridecylether ratio 1:2 w/w%) induced a more intense and statistically significant itching decrease than the placebo shampoo SL15VS021-1after 1, 5, 15 and 30 minutes from the first product use. **Table 3** and **Figure 1** provide the results of the short term evaluation of itching effect of shampoos SL15VS021-2 (comprising 2% of PEG-5 Isononanoate/PEG-9 Tridecylether ratio 1:2 w/w%) and shampoo SL15VS021-1 (placebo); data received after 1, 5, 15 and 30 minutes.

**Table 3**

| Short term evaluation of itching effect of shampoos | | | |
|---|---|---|---|
| **Itching decrease** | **SL15VS021-2 2% of PEG-5 Isononanoate/PEG-9 Tridecylether ratio 1:2 w/w%** | **SL15VS021-1 Placebo** | **Wilcoxon Test SL15VS021-2 vs SL15VS021-1 p-level** |
| after 1 minutes | mean 1.2 | mean 0.4 | p < 0.05 |
| | *std. dev. 1.0* | *std. dev. 0.5* | |
| after 5 minutes | mean 1.3 | mean 0.4 | p < 0.01 |
| | *std. dev. 1.0* | *std. dev. 0.5* | |
| after 15 minutes | mean 1.6 | mean 0.1 | p < 0.001 |
| | *std. dev. 0,9* | *std. dev. 0.3* | |
| after 15 minutes | mean 1.4 | mean 0.1 | p < 0.01 |
| | std. dev. 1.0 | std. dev. 0.3 | |

### Results of long term evaluation

The clinical evaluation evidenced that the use of the shampoo SL15VS021-2 (with 2% of PEG-5 Isononanoate/PEG-9 Tridecylether ratio 1:2 w/w %, induced a more and statistically significant decrease in erythema and itching sensation in comparison to the placebo shampoo SLVS021-1. No statistically significant variation was found for dandruff, seborrhoea and burning sensation. The product neither had negative effects nor induced any irritation on the scalp; it was very well tolerated on the whole by all volunteers. The results for long term evaluation of anti-itch shampoos SL15VS021-2 (comprising 2% of PEG-5 Isononanoate/PEG-9 Tridecylether ratio 1:2 w/w%, after 7 days repeated application, are compiled in **Tables 4 to 6** and **Figure 2****.**

**Table 4**

| Short term evaluation of itching effect of shampoos | | | |
|---|---|---|---|
| **SL15VS021-2 2% of PEG-5 Isononanoate/PEG-9 Tridecylether ratio 1:2 w/w%** | **T₀** | **T_{7days}** | **Wilcoxon Test T_{0 vs} T_{7days} p-level** |
| Dandruff | mean 0.6 | mean 0.6 | p > 0.05 |
| | *std. dev. 0,8* | *std. dev. 0.8* | |
| Seborrhoea | mean 1.7 | mean 1.6 | p > 0.05 |
| | *std. dev. 0.8* | *std. dev. 0,9* | |
| Erythema | mean 1,2 | mean 0.7 | p < 0.01 |
| | *std. dev. 1,0* | *std. dev. 0.9* | |
| Itching | mean 1.9 | mean 0,8 | p < 0.001 |
| | *std. dev. 0,7* | *std. dev. 0.8* | |
| Burning | mean 0.3 | mean 0 | p > 0.05 |
| | *std. dev. 0.7* | *std. dev. 0* | |
| Tolerability | - | mean 3,0 | |
| | | *std. dev. 0* | |

**Table 5**

| Long term evaluation of shampoo SL15VS021-1 (placebo) after 7 days repeated application | | | |
|---|---|---|---|
| **SL15VS021-1 (Placebo)** | **T₀** | **T_{7days}** | **Wilcoxon Test T_{0 vs} T_{7days} p-level** |
| Dandruff | mean 0.6 | mean 0.6 | p > 0.05 |
| | *std. dev. 0,8* | *std. dev. 0.8* | |
| Seborrhoea | mean 1.7 | mean 1.8 | p > 0.05 |
| | *std. dev. 0.9* | *std. dev. 0,9* | |
| Erythema | mean 1,3 | mean 1,3 | p > 0.05 |
| | *std. dev. 1,0* | *std. dev. 1,0* | |
| Itching | mean 1.8 | mean 1,5 | **p < 0.05** |
| | *std. dev. 0,6* | *std. dev. 0.7* | |
| Burning | mean 0.3 | mean 0,3 | p > 0.05 |
| | *std. dev. 0.7* | *std. dev. 0,7* | |
| Tolerability | - | mean 3,0 | |
| | | *std. dev. 0* | |

**Table 6**

| Statistical comparison between the variations for SL15VS021-2 vs SL15VS021-1 | |
|---|---|
| **SL15VS021-2 (2% PEG-5 Isononanoate/PEG-9 Tridecylether ratio 1:2 w/w%, vs SL15VS021-1 (placebo)** | **Wilcoxon test T_{0 vs} T_{7days} p-level** |
| Dandruff | p > 0.05 |
| Seborrhoea | p > 0.05 |
| Erythema | p < 0.01 |
| Itching | p < 0.01 |
| Burning | p > 0.05 |

The clinical evaluation evidenced that the use of the shampoo SL15VS021-2 (with 2% PEG-5 Isononanoate/PEG-9 Tridecylether ratio 1:2 w/w %) induced a statistically significant decrease in erythema and in itching sensation in comparison to the placebo after 7 days repeated application.

Furthermore, no significant variation among the set of data was found for dandruff, seborrhoea, and burning sensation. This proved that the use of the products neither had negative effects nor induced any irritation on the skin site.

Referring to immediate and instant itching decrease, shampoo SL15VS021-2 (with 2% PEG-5 Isononanoate/PEG-9 Tridecylether ratio 1:2 w/w %) induced a more intense and statistically significant variation than the Placebo shampoo after 1, 5, 15 and 30 minutes from the first product use.

### FORMULATION EXAMPLES 1 TO 15

Formulations (compositions) comprising compounds according to formula 1 having (skin soothing) itch-reducing action:
**1.** Skin lightening day cream o/w
**2:** Skin-soothing lotion
**3:** After sun balm, itch reducing
**4:** Calming body spray
**5:** Sunscreen lotion (o/w, broadband protection)
**6:** w/o night cream
**7:** Scalp soothing Anti dandruff shampoo
**8:** Self-tanning cream
**9:** Anti itch barrier repair cream
**10:** Antiperspirant/deodorant roll-on
**11:** Emulsion with UV-A/B-broadband protection
**12:** Sun spray with UV-A/B-broadband protection with low oil content
**13:** Skin-lightening balm with UV-A/UV-B protection
**14:** Scalp soothing hair conditioner with UV-B/UV-A protection, rinse off
**15:** Anti-itch hair conditioner, leave on

| **RAW MATERIAL NAME/INCI** | **INCI** | **% BY WEIGHT / FORMULATION EXAMPLE** | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| PEG-5 Isononanoate /PEG-9 Tridecylether ratio 1:2 w/w % | | 0.3 | 1.0 | 0.5 | 3.0 | 0.5 | 2.0 | 0.1 | 0.0 5 | 5.0 | 2.0 | 1.0 | 1.0 | 0.6 | 1.5 | 5.0 |
| Abil 350 | Dimethicone | 0.5 | 2.0 | 1.0 | | | | | 0.5 | 0.5 | | 0.3 | | | 0.1 | |
| Allantoin | Allantoin | | 0.2 | 0.1 | | | | | | 0.2 5 | | | | | | |
| Aloe Vera Gel Concentrate 10/1 * | Water (Aqua). Aloe Barbadensis Leaf Juice | | | 3.0 | | | 3.0 | 0.4 5 | | | | | | | | |
| Alpinia Leaf Extract Blend | Alpinia Officinarum Leaf Extract. Alpinia conchigera Leaf Extract. Alpinia Blepharocalyx Leaf Extract | | | | | 1.0 | | | | | 0.5 | | | | | |
| Alugel 34 TH | Aluminium Stearate | | | | | | 1.0 | | | | | | | | | |
| Arbutin | β-Arbutin | 0.2 | | | | | | | | | | | | | | |
| Butylene Glycol | Butylene Glycol | | | 5.0 | | | | | | | | 3.0 | | 3.0 | | |
| Carbopol Ultrez-10 | Carbomer | | 0.1 | | | 0.2 | | | | | | 0.2 | | | | |
| Ceramide BIO* | Cetylhydroxyproline Palmitamide | | 0.1 | | | | | 0.2 | | 0.5 | | | | | | |
| Cetiol OE | Dicaprylyl Ether | | | 4.0 | | | | | | | | | | | | |
| Cetiol SB 45 | Butyrospermum Parkii (Shea Butter) | | | 1.0 | | | | | | | | | | | | |
| Citric Acid 10% sol. | Citric Acid | | 0.4 | | | | | 0.3 | | | | 0.3 | | | | |
| Comperlan 100 | Cocamide MEA | | | | | | | 0.5 | | | | | | | | |
| Crinipan AD | Climbazole | | | | | | | 0.5 | | | | | | | | |
| Curcuma Extract | Curcuma Xanthorrhiza Root Extract | | | | | | | | | | 0.5 | | | | | |
| Curcuma Root Extract | Curcuma Longa (Turmeric) Root Extract | | 1.5 | | | | | | | | | | | | | |
| Dehyquart A CA | Cetrimonium Chloride | | | | | | | | | | | | | | 0.2 | 0.5 |
| Dehyquart SP | Quaternium-52 | | | | | | | | | | | | | | 0.5 | 4.0 |
| Dihydroxyacet one | Dihydroxyacetone | | | | | | | | 5.0 | | | | | | | |
| Dow Corning 246 Fluid | Cyclohexasilox-ane and Cyclopentasilox-ane | | | | | 2.0 | | | | | | | | | | |
| Dow Corning 345 Fluid | Cyclomethicone | | | | 0.5 | | | | | | | | | | | |
| D-Panthenol | Panthenol | | | 1.0 | | | | | | | | | | | | |
| Dracorin® CE* | Glyceryl Stearate Citrate | 5.0 | | | | | | | 5.0 | 1.5 | | | | | 1.0 | 1.0 |
| Dracorin® GOC* | Glyceryl Oleate Citrate. Caprylic/Capric Triglyceride | | | | 2.0 | | | | | | | | | | | |
| Drago-Beta-Glucan* | Water (Aqua). Butylene Glycol. Glycerin. Avena Sativa (Oat). Kernel Extract | 0.3 | | | | | | | | | | | | | | |
| Dragoderm® * | Glycerin. Triticum Vulgare (Wheat) Gluten. Water (Aqua) | | | | | | | 2.0 | | | | | | | | |
| Drago-Oat-Active* | Water (Aqua). Butylene Glycol. Avena Sativa (Oat) Kernel Extract | | | | 1.0 | | | | | | | | | | | 2.0 |
| Dragosan W/O P* | Sorbitan Isostearate. Hydrogenated Castor Oil. Ceresin. Beeswax (Cera Alba) | | | | | | 6.0 | | | | | | | | | |
| Dragosantol® 100* | Bisabolol | 0.3 | | | 0.1 | 0.3 | 0.2 | | | | 0.1 | 0.1 | | | | |
| Dragoxat® 89 * | Ethylhexyl Ethylisononan-oate | | | | | | | | | 2.0 | | | | 0.1 | | |
| EDETA BD | Disodium EDTA | | | | | 0.1 | | | 0.1 | | | 0.1 | | | 0.1 | |
| Emulsiphos ® * | Potassium Cetyl Phosphate. Hydrogenated Palm Glycerides | | 2.0 | | | 1.5 | | | | 2.0 | | 1.5 | | 0.1 | | |
| Ethanol 96 % | Ethanol | | | | | | | | 2.0 | | 30.0 | | 13. 0 | 5.0 | | |
| Extrapone® Green Tea GW * | Glycerin. Water (Aqua). Camellia Sinensis Leaf Extract | | 0.2 | | | | | | | | | | | | 0.7 | |
| Extrapone® Rosemary GW* | Glycerin. Water (Aqua). Rosmarinus officinalis (Rosemary) Leaf Extract | | 0.3 | | | | | | | 0.5 | | | | | | |
| Extrapone® Witch Hazel Distillate colourless* | Propylene Glycol. Hamamelis Virginiana (Witch Hazel) Water. Water (Aqua). Hamamelis Virginiana (Witch Hazel) Extract | | | | | | 1.0 | | | | | | | | | |
| Farnesol* | Farnesol | | | | | | | | | | 0.5 | | | | | |
| Fragrance "Rose"* | Fragrance | | | | | | | | | | | 0.3 | | | | |
| Fragrance "WHITE" * | Fragrance | 0.3 | 0.3 | 0.3 | 0.2 | 0.4 | 0.4 | 0.5 | 0.3 | 0.3 | 1.0 | | 0.5 | 0.4 | 0.5 | 0.1 |
| Frescolat® MG A* | Menthone Glycerol Acetal | 0.5 | | | | 0.3 | | | | | | | | | | |
| Frescolat® ML cryst.* | Menthyl Lactate | | | 0.8 | | | | | | | 0.2 | | | | | |
| Frescolat® X-COOL* | Menthyl Ethylamido Oxalate | | | | | | | | | | | 1.0 | | | | |
| Genapol LRO liquid | Sodium Laureth Sulfate | | | | | | | 37. 0 | | | | | | | | |
| Glycerol 85 % | Glycerin | 3.0 | 2.0 | 4.0 | | 4.7 | 2.0 | | 1.5 | 3.0 | | | | | | |
| Glyceryl Stearate | Glyceryl Stearate | | 2.0 | | | | | | | 2.0 | | 2.0 | 4.0 | | | |
| Hydrolite® -5* | Pentylene Glycol | | | | 5.0 | | | | 3.5 | | | | | | | |
| Hydroviton® 24* | Water. Glycerin. Sodium Lactate. TEA Lactate. Serine. Lactic Acid. Urea. Sorbitol. Sodium Chloride. Lauryl Diethylenedi-aminoglycine. Lauryl Aminopropyl-glycine. Allantoin | | | | | | | | | 1.0 | | | | 4.5 | | |
| Hydroviton® PLUS* | Water. Pentylene Glycol. Glycerin. Fructose. Urea. Citric Acid. Sodium Hydroxide. Maltose. Sodium PCA. Sodium Chloride. Sodium Lactate. Trehalose. Allantoin. Sodium hyaluronate. Glucose | | | 1.0 | | | | | | | | | 1.0 | | | |
| Irgasan DP 300 | Triclosan | | | | | | | | | | 0.3 | | | | | |
| Isoadipate® * | Diisopropyl Adipate | | | | | | | | | | | | 1.0 | 1.0 | | |
| Isodragol® * | Triisononanoin | | 2.0 | | | | | | | 3.0 | | | | 1.0 | | |
| Isopropyl Palmitate | Isopropyl Palmitate | 4.0 | | | | | | | 4.0 | | | | | | | |
| Karion F | Sorbitol | | | | | | 2.0 | | | | | | | | | |
| Keltrol RD | Xanthan Gum | 0.2 | 0.1 | | | 0.2 | | | 0.3 | | | 0.2 | | | | |
| Kojic acid | Kojic Acid | 1.0 | | | | | | | | | | 0.5 | 0.2 | 0.3 | | |
| Lanette 16 | Cetyl Alcohol | 1.0 | | | | | | | 1.0 | | | 1.2 | | | | |
| Lanette E | Sodium Cetearyl Sulfate | | | | | | | | | | | 0.7 | | | | |
| Lanette O | Cetearyl Alcohol | | 3.0 | | | 1.0 | | | | 2.0 | | | | | | |
| Lara Care A-200 | Galactoarabinan | | | 0.3 | | | | | | | | | | | 2.5 | 1.5 |
| Magnesium Chloride | Magnesium Chloride | | | | | | 0.7 | | | | | | | | | |
| Merquat 550 | Polyquaternium-7 | | | | | | | 0.5 | | | | | | | | |
| NaOH 10% sol. | Sodium Hydroxide | | | | | | | | | 0.3 | | | | | | |
| Naringin | 4'.5.7-Trihydroxyflavone7-O-Neohesperidoside | | | | | | | 0.5 | 2.0 | | | | | | | |
| Natrosol 250 HHR | Hydroxyethyl-cellulose | | | | | | | | | | 0.3 | | | | | |
| Neo Heliopan® 357* | Butyl Methoxydibenzoyl-methane | | | | | 1.0 | | | | | | | | | | |
| Neo Heliopan® AP* (10 % as sodium salt) | Disodium Phenyl Dibenzimidazole Tetrasulfonate | | | | | 10 | | | | | | 22. 0 | | 1.5 | | |
| Neo Heliopan® AV* | Ethylhexyl Methoxycinnamate | 5.0 | | | | 3.0 | | | | | | | 25. 0 | | | |
| Neo Heliopan® E1000* | Isoamyl p-Methoxycinnamate | | | | | | | | | | | | | 5.0 | | |
| Neo Heliopan® HMS* | Homosalate | | | | | | | | | | | 5.0 | | 5.0 | | |
| Neo Heliopan® Hydro* (15 % as sodium salt) | Phenylbenz-imidazole Sulfonic Acid | | | | | 6.7 | | | | | | | | | | |
| Neo Heliopan® MBC* | 4-Methylbenzyl-idene Camphor | | | | | 1.5 | | | | | | | 33. 3 | 10. 0 | | |
| Neo Heliopan® OS* | Ethylhexyl Salicylate | | | | | 5.0 | | | | | | | | 2.0 | | |
| Neo PCL wssl. N* | Trideceth-9. PEG-5 Ethylhexanoate. Water | | | | | | | | | | | | | | | |
| Neutral Oil | Caprylic/Capric Triglyceride | 6.0 | | | 4.0 | 2.0 | | | 6.0 | 10. 0 | | 2.0 | | | | 1.0 |
| Oxynex 2004 | BHT | | | | | | 0.1 | | | | | | | | | |
| Paraffin Oil | Mineral Oil | | | | 4.0 | | | | | | | | | | | |
| PCL Liquid 100* | Cetearyl Ethylhexoate | 3.0 | 5.0 | | 7.0 | | 12. 0 | | 3.0 | | | 3.0 | | 0.6 | | 0.3 |
| PCL Solid* | Stearyl Heptanoate. Stearyl Caprylate | | 2.0 | | | | | | | | | | | | 3.0 | |
| Pemulen TR-2 | Acrylates/C10-30 Alkyl Acrylate Crosspolymer | | | 0.3 | 0.2 | | | | | | | | | | | |
| Polymer JR 400 | Polyquaternium-10 | | | | | | | | | | | | | | | 0.1 |
| Propylene Glycol | Propylene Glycol | | 5.0 | | | | | | | | | | 0.8 | 0.8 | | 0.8 |
| Retinyl Palmitate in Oil | Retinyl Palmitate | | | | | | 0.2 | | | | | | | | | |
| Sepigel 305 | Polyacrylamide. C13-14 Isoparaffin. Laureth-7 | | | | | | | | 1.0 | | | | | | | |
| Sodium Ascorbyl Phosphate | Sodium Ascorbyl Phosphate | 2.0 | | 1.0 | | | | | | | | | | | | |
| Sodium Benzoate | Sodium Benzoate | | | | | | | 0.5 | | | | | | | | |
| Sodium Chloride | Sodium Chloride | | | | | | | 1.0 | | | | | | | | |
| Sodium Hydroxide (10% sol.) | Sodium Hydroxide | | 0.3 | 0.6 | 0.4 | | | | | | | 2.8 | | | | |
| Solubilizer 611674* | PEG-40 Hydrogenated Castor Oil. Trideceth-9. Water (Aqua) | | | | | | | | | | 2.0 | | | 2.2 | | |
| Sun Flower Oil | Helianthus Annuus (Sunflower) Seed Oil | | | | | | 5.0 | | | | | | | | | |
| Sweet Almond Oil | Prunus dulcis | | | | | | 5.0 | | | | | | | | | |
| SymCalmin® | Pentylene Glycol. Butylene Glycol. Hydroxyphenyl Propamidobenzoic Acid | | | 1.0 | | | | | | 1.0 | | | | | | |
| SymDeo® B125* | 2-Methyl 5-Cyclohexylpentanol | | | | | | | | | | 0.5 | | | | | |
| SymDeo® MPP* | Dimethyl Phenylbutanol | | | | 0.5 | | | | | | | | | | | |
| Symdiol® 68* | 1.2-Hexanediol. Caprylylglycol. | | | | | | | | | | | 0.5 | | | | |
| Symdiol® 68T* | 1.2-Hexanediol. Caprylylglycol. Tropolone | 0.5 | | | | | | | | | | | | | | |
| SymMatrix® * | Maltodextrin. Rubus Fruticosus (Blackberry) Leaf Extract | | 0.1 | | | 0.3 | 1.0 | | | | | | | | | |
| SymMollient® S * | Cetearyl Nonanoate | | | | | | | | | | | 1.5 | | | | |
| SymOcide® PS * | Phenoxyethanol. Decylene Glycol. 1.2 Hexanediol | | | | | | | 1.0 | | | | | | | | |
| Polyoxyethyle ne (9) Lauryl Ether | Laureth-9 | | 0.5 | | | | | | | | | | | | 1.0 | |
| SymRelief® 100* | Bisabolol. Zingiber Officinale (Ginger) Root Extract | | | | 0.1 | | | | | | | | | | | |
| SymRepair® 100* | Hexyldecanol. Bisabolol. Cetylhydroxyproline Palmitamide. Stearic Acid. Brassica Campestris (Rapeseed) Sterols | | | | | | | | | 2.0 | | | | | | |
| SymSitive® 160 9 * | Pentylene Glycol. 4-t-Butylcyclohexanol | | | 1.5 | | | | | | | | 0.5 | | | | |
| SymSol®PF3 * | Water. Pentylene Glycol. Sodium Lauryl Sulfoacetate. Sodium Oleoyl Sarcosinate. Sodium Chloride. Disodium Sulfoacetate. Sodium Oleate. Sodium Sulfate | | | | | | | | | | | | | | | 1.5 |
| SymVital AgeRepair* | Zingiber Officinale (Ginger) Root Extract | 0.1 | | | | | 0.1 | | | | | | | | | |
| SymWhite® 37 7* | Phenylethyl Resorcinol | 0.5 | | | | | | | | | | 0.5 | | 1.0 | | |
| Tego Betain L7 | Cocamidopropyl Betaine | | | | | | | 6.0 | | | | | 1.0 | 1.0 | | |
| Tegosoft PC 31 | Polyglyceryl 3- Caprate | | | | | | | | | 0.3 | | | | | | |
| Tegosoft TN | C12-15 Alkyl Benzoate | | | 5.0 | | 5.0 | | | | | | | | | | |
| Texapon NSO BZ | Sodium Laureth Sulfate | | | | | | | | | | | | | 4.0 | | |
| Tocopherol Acetate | Tocopheryl Acetate | | | 0.5 | | 0.5 | 3.0 | | | 0.3 | | 0.5 | | | | |
| Triethanolami ne. 99% | Triethanolamine | | | | | 0.5 | | | | | | | | 0.5 | | |
| Water. demineral ized | Water (Aqua) | ad 100 | | | | | | | | | | | | | | |
| Zedoaria Leaf Extract | Curcuma Zedoaria Leaf Extract | 2.0 | | 1.5 | | | | | | | | | | | | |
| Zirkonal L 450 | Aluminium Zirconium Pentachloro-hydrate (40 % aqueous solution) | | | | | | | | | | 37.0 | | | | | |

## Claims

1. A pharmaceutical composition comprising or consisting of at least one polyalkylene glycol derivative according to general formula (I)
**R¹(OCH₂CHR²)ₙOR³** **(I)**
in which
R¹ stands for hydrogen or a linear or branched alkyl group with 1 to 4 carbon atoms;
R² stands for hydrogen or methyl;
n represents an integer of from about 3 to about 20
R³ stands for a linear or branched alkyl or alkenyl group with 6 to 18 carbon atoms and 0 or 1 to 3 double bonds or a -COR⁴ acyl group; and
R⁴ stands for hydrogen, a linear or branched alkyl or alkenyl radical having 5 to 17 carbon atoms, or an alkali metal, an alkaline earth metal or ammonia
for use in the prevention, treatment and/or cure of itchy conditions of human skin and scalp.

2. The composition for use of Claim 1 comprising or consisting of at least one polyalkylene glycol derivative according to formula (Ia)
**H(OCH₂CH₂)ₙOR³** **(Ia)**
in which n represents an integer of from about 7 to 12 and R³ stands for a linear or branched alkyl group with 8 to 15 carbon atoms.

3. The composition for use of Claim 1 comprising or consisting of at least one polyalkylene glycol derivative according to formula (Ib)
**H(OCH₂CH₂)ₙO-COR⁴** **(Ib)**
in which n represents an integer of from about 3 to about 7 and R⁴ stands for a linear or branched alkyl group with 7 to 11 carbon atoms.

4. The composition for use of Claim 2 comprising or consisting of PEG-9 Tridecylether.

5. The composition for use of Claim 3 comprising or consisting of PEG-5 Isononanoate.

6. The composition for use of Claim 1 comprising or consisting of
(a) at least one polyalkylene glycol derivative according to formula (Ia) and
(b) at least one polyalkylene glycol derivative according to formula (Ib).

7. The composition for use of Claim 6, comprising or consisting of a mixture of
(a) PEG-9 Tridecylether and
(b) PEG-5 Isononanoate.

8. The composition for use of Claim 6 or Claim 7, wherein said components (a) and (b) are present in a ratio by weight of about 1:5 to 5:1.

## Patentansprüche

1. Pharmazeutische Zubereitung, enthaltend oder bestehend aus wenigstens einem Polyalkylenglykolderivat gemäß der allgemeinen Formel (I),
**R¹(OCH₂CHR²)ₙOR³** **(I)**
in der
R¹ für Wasserstoff oder eine lineare oder verzweigte Alkylgruppe mit 1 bis 4 Kohlenstoffatomen steht;
R² für Wasserstoff oder Methyl steht;
n für eine ganze Zahl von etwa 3 bis etwa 20 steht,
R³ für eine lineare oder verzweigte Alkyl- oder Alkenylgruppe mit 6 bis 18 Kohlenstoffatomen und 0 oder 1 bis 3 Doppelbindungen oder eine -COR4-Gruppe steht, und
R⁴ für Wasserstoff, ein lineares oder verzweigtes Alkyl- oder Alkenylradikal mit 5 bis 17 Kohlenstoffatomen, oder ein Alkalimetall, ein Erdalkalimetall oder Ammonium steht,
zur Verwendung in der Vorbeugung, Behandlung und/oder Heilung von Juckzuständen von Haut und Haaren.

2. Zusammensetzung zur Verwendung gemäß Anspruch 1, enthaltend oder bestehend aus wenigstens einem Polyalkylenglykolderivat gemäß der Formel (I),
**H(OCH₂CH₂)ₙOR³** **(Ia)**
in der n für eine ganze Zahl von etwa 7 bis etwa 12 und R³ für eine lineare oder verzweigte Alkylgruppe mit 8 bis 15 Kohlenstoffatomen steht.

3. Zusammensetzung zur Verwendung nach Anspruch 1, enthaltend oder bestehend aus mindestens einem Polyalkylenglykolderivat der Formel (Ib)
**H(OCH₂CH₂)ₙO-COR⁴** **(Ib)**
in der n eine ganze Zahl von etwa 3 bis etwa 7 und R4 für eine lineare oder verzweigte Alkylgruppe mit 7 bis 11 Kohlenstoffatomen steht.

4. Zubereitung zur Verwendung nach Anspruch 2, enthaltend oder bestehend aus PEG-9 Tridecylether.

5. Zubereitung zur Verwendung nach Anspruch 3, enthaltend oder bestehend aus PEG-5 Isononanoat.

6. Zubereitung zur Verwendung nach Anspruch 1, enthaltend oder bestehend aus
(a) wenigstens einem Polyalkylenglykolderivat der Formel (Ia) und
(b) wenigstens einem Polyalkylenglykolderivat der Formel (Ib).

7. Zubereitung zur Verwendung nach Anspruch 6, enthaltend oder bestehend aus einer Mischung aus
(a) PEG-9 Tridecylether und
(b) PEG-5 Isononanoat.

8. Zubereitung zur Verwendung nach den Ansprüchen 6 oder 7, wobei die Komponenten (a) und (b) in einem Gewichtsverhältnis von etwa 1:5 bis etwa 5:1 vorliegen.

## Revendications

1. Composition pharmaceutique comprenant ou constituée d'au moins un dérivé de polyalkylène glycol répondant à la formule générale (I)
**R¹(OCH₂CHR²)ₙOR³** **(I)**
dans laquelle
R¹ désigne un atome d'hydrogène ou un groupement alkyle linéaire ou ramifié comportant 1 à 4 atomes de carbone ;
R² désigne un atome d'hydrogène ou un groupement méthyle ; n représente un entier compris entre environ 3 et environ 20
R³ désigne un groupement alkyle ou alcényle linéaire ou ramifié comportant 6 à 18 atomes de carbone et 0 ou 1 à 3 doubles liaisons ou un groupement acyle en-COR⁴; et
R⁴ désigne un atome d'hydrogène ou un radical alkyle ou alcényle linéaire ou ramifié comportant 5 à 17 atomes de carbone, ou un métal alcalin, un métal alcalino-terreux ou l'ammoniac
pour utilisation dans le traitement prophylactique ou thérapeutique et/ou la guérison d'états de démangeaison de la peau et du cuir chevelu humains.

2. Composition pour utilisation selon la Revendication 1 comprenant ou constituée d'au moins un dérivé de polalkylène glycol répondant à la formule (la)
**H(OCH₂CH₂)ₙOR³** **(Ia)**
dans laquelle n représente un entier compris entre environ 7 et 12 et R³ désigne un groupement alkyle linéaire ou ramifié comportant entre 8 et 15 atomes de carbone.

3. Composition pour utilisation selon la Revendication 1 comprenant ou constituée d'au moins un dérivé de polalkylène glycol répondant à la formule (Ib)
**H(OCH₂CH₂)ₙO-COR⁴** **(Ib)**
dans laquelle n représente un entier compris entre environ 3 et environ 7 et R⁴ désigne un groupement alkyle linéaire ou ramifié comportant entre 7 et 11 atomes de carbone.

4. Composition pour utilisation selon la Revendication 2 comprenant ou constitué de PEG-9 Tridécyléther.

5. Composition pour utilisation selon la Revendication 3 comprenant ou constitué de PEG-5 Isononanoate.

6. Composition pour utilisation selon la Revendication 1 comprenant ou constitué
(a) d'au moins un dérivé de polyalkylène glycol répondant à la formule (la) et
(b) d'au moins un dérivé de polyalkylène glycol répondant à la formule (Ib).

7. Composition pour utilisation selon la Revendication 6 comprenant ou constitué d'un mélange de
(a) PEG-9 Tridécyléther et
(b) PEG-5 Isononanoate.

8. Composition pour utilisation selon la Revendication 6 ou la Revendication 7, où lesdits composants (a) et (b) sont présents dans un rapport massique compris entre environ 1:5 et 5:1.
